(19) Europäisches Patentamt

European Patent Office

Office européen des brevets

(11) **EP 2 083 684 B1**

(12) **FASCICULE DE BREVET EUROPEEN**

(45) Date de publication et mention
de la délivrance du brevet:
**05.12.2018 Bulletin 2018/49**

(21) Numéro de dépôt: **07822549.7**

(22) Date de dépôt: **13.11.2007**

(51) Int Cl.:
***A61B 5/103*** *(2006.01)*    ***A61F 2/68*** *(2006.01)*
***A61F 5/01*** *(2006.01)*

(86) Numéro de dépôt international:
**PCT/EP2007/062280**

(87) Numéro de publication internationale:
**WO 2008/058966 (22.05.2008 Gazette 2008/21)**

(54) **DISPOSITIF ET PROCÉDÉ DE SUIVI DU MOUVEMENT D'UN ÊTRE VIVANT**

VORRICHTUNG UND VERFAHREN ZUR VERFOLGUNG DER BEWEGUNG EINES LEBEWESENS

DEVICE AND METHOD FOR TRACKING THE MOVEMENTS OF A LIVE BEING

(84) Etats contractants désignés:
**AT BE BG CH CY CZ DE DK EE ES FI FR GB GR
HU IE IS IT LI LT LU LV MC MT NL PL PT RO SE
SI SK TR**

(30) Priorité: **15.11.2006 FR 0654922**

(43) Date de publication de la demande:
**05.08.2009 Bulletin 2009/32**

(73) Titulaire: **Commissariat à l'Énergie Atomique
et aux Énergies Alternatives
75015 Paris (FR)**

(72) Inventeurs:
 • **HELIOT, Rodolphe
 64600 Anglet (FR)**
 • **AZEVEDO-COSTE, Christine
 34560 Montbazin (FR)**

(74) Mandataire: **Ilgart, Jean-Christophe et al
BREVALEX
95, rue d'Amsterdam
75378 Paris Cedex 8 (FR)**

(56) Documents cités:
**FR-A- 2 885 434        US-A1- 2004 088 057
US-A1- 2005 197 717**

 • **REHBINDER H ET AL: "Drift-free attitude
estimation using quasi-linear observers" NEW
DIRECTIONS AND APPLICATIONS IN CONTROL
THEORY SPRINGER-VERLAG BERLIN,
GERMANY, 2005, pages 321-336, XP008088551
ISBN: 3-540-23953-7**
 • **ZIELINSKA T: "COUPLED OSCILLATORS
UTILISED AS GAIT RHYTHM GENERATORS OF
A TWO-LEGGED WALKING MACHINE"
BIOLOGICAL CYBERNETICS, SPRINGER
VERLAG, HEIDELBERG, DE, vol. 74, no. 3, 1996,
pages 263-273, XP000553469 ISSN: 0340-1200**
 • **DUTRA M S ET AL: "Modeling of a bipedal
locomotor using coupled nonlinear oscillators of
Van der Pol" BIOLOGICAL CYBERNETICS
SPRINGER-VERLAG GERMANY, vol. 88, no. 4,
avril 2003 (2003-04), pages 286-292, XP002441605
ISSN: 0340-1200**
 • **VERDAASDONK ET AL: "Energy efficient and
robust rhythmic limb movement by central
pattern generators" NEURAL NETWORKS,
ELSEVIER SCIENCE PUBLISHERS, BARKING,
GB, vol. 19, no. 4, mai 2006 (2006-05), pages
388-400, XP005495122 ISSN: 0893-6080**

Il est rappelé que: Dans un délai de neuf mois à compter de la publication de la mention de la délivrance du brevet
européen au Bulletin européen des brevets, toute personne peut faire opposition à ce brevet auprès de l'Office européen
des brevets, conformément au règlement d'exécution. L'opposition n'est réputée formée qu'après le paiement de la taxe
d'opposition. (Art. 99(1) Convention sur le brevet européen).

**Description**

Le sujet de cette invention est le suivi du mouvement d'un être vivant afin de le reproduire ou de le compléter ; une application envisagée aujourd'hui, mais qui n'est pas exclusive puisque beaucoup d'autres sont à envisager, est l'aide à la marche d'une personne à mobilité réduite, hémiplégique et donc possédant une seule jambe valide, ou d'une personne possédant une prothèse de jambe.

On cherche alors à faire effectuer à la jambe invalide un mouvement complétant celui de la jambe valide pour donner une démarche aussi normale que possible. Une commande artificielle de mouvement d'une jambe invalide peut prendre plusieurs formes : chez le patient hémiplégique, on peut produire une stimulation électrique fonctionnelle sur les muscles de la jambe invalide à l'aide d'électrodes afin de les contracter et de créer ainsi le mouvement ; sur une prothèse, on peut faire une régulation du mouvement du genou artificiel, soit en le bloquant dans les phases où la personne qui en est équipée s'appuie sur lui, soit au contraire en le laissant très souple dans les phases où il ne doit pas freiner le mouvement.

Il est alors tentant d'utiliser des capteurs de mouvements sur la jambe valide et de traiter leurs signaux de façon appropriée pour obtenir des instructions de commande du dispositif artificiel placé sur la jambe invalide. Les articles de Williamson (Williamson, R.p.; Andrews, B.J.; Au, R.: "Control of neural prostheses. II. Event détection using machine learning "Proceedings of the RESNA '96 Annual Conférence Exploring New Horizons... Pioneering the 21st Century, p. (291-3), 1996) et Pappas (Pappas I.P.I.; Keller, T.; Mangold, S.: "A reliable, gyroscope base gait phase détection sensor embedded in a shoe insole" Proceedings of IEEE Sensors 002. First IEEE International Conférence on Sensors, p. 1085-8 vol.2, 2002) indiquent quelques façons de procéder. Des états discrets de la marche sont détectés sur les signaux des capteurs et permettent de fournir une commande de la jambe invalide, mais pas de coordonner parfaitement les mouvements des deux jambes. Un art antérieur analogue apparaît dans US-A- 2004/088057. Des modèles numériques de création de mouvement cyclique ont aussi été proposés, avant tout dans le domaine de la robotique pour reproduire, par exemple, une démarche. Ces travaux, conçus pour animer des corps artificiels, ne permettent pas de coordonner ou de synchroniser de façon satisfaisante un membre naturel et une membre artificiel dans des situations de démarche variable.

REHBINDER H ET AL: "Drift-free attitude estimation using quasi-linear observers" NEW DIRECTIONS AND APPLICATIONS IN CONTROL THEORY, SPRINGER-VERLAG BERLIN, GERMANY, 2005, pages 321-336, ISBN: 3-540-23953-7, décrit un observateur numérique pour suivre le mouvement d'un robot mobile.

L'objet de l'invention est donc de perfectionner les procédés existants pour obtenir une coordination ou une synchronisation meilleure des mouvements des deux jambes. Elle recourt à un modèle numérique, enregistré au préalable, de la démarche, mais que le contrôleur engendrant la démarche sur la jambe artificielle adapte sans cesse à la démarche réelle grâce à un observateur numérique qui suit à la fois le modèle et des signaux des capteurs, qu'il interprète pour en déduire les commandes à appliquer.

Un aspect de l'invention est un dispositif de suivi de mouvement d'un être vivant, comprenant un capteur fixé à l'être vivant et recueillant des signaux représentatifs de son mouvement, et un contrôleur artificiel de traitement des signaux du capteur, où le contrôleur artificiel comprend un module de calcul, caractérisé en ce que le module de calcul comprend un observateur numérique apte à délivrer un signal de sortie d'après un modèle de référence du mouvement enregistré dans le contrôleur artificiel, le signal de sortie étant fonction des signaux du capteur et du modèle de référence, le modèle de référence et le signal de sortie étant exprimés en variables d'état du mouvement relatives à une phase du mouvement.

Un autre aspect de l'invention concerne un procédé de suivi de mouvement d'un être vivant apte à accomplir seulement une portion effective du mouvement, en un procédé de suivi du mouvement d'un être vivant, consistant à mesurer continûment une portion effective du mouvement par un capteur fixé sur l'être vivant, à utiliser des signaux du capteur de façon à estimer des valeurs de variables d'état du mouvement pour produire des instructions de commande et entraîner un dispositif accomplissant une portion complémentaire du mouvement, caractérisé en ce qu'il consiste à utiliser aussi un modèle de référence, obtenu dans une étape d'étalonnage et défini par des séries de valeurs prises par les variables d'état du mouvement à des phases du mouvement, pour estimer les valeurs des variables d'état du mouvement.

Une bonne façon de résoudre numériquement l'ajustement est obtenue si l'observateur numérique effectue continûment un ajustement d'un paramètre d'après des mesures du capteur en résolvant numériquement une équation différentielle, et un ajustement d'au moins une des variables d'état d'après le paramètre intermédiaire.

Dans ce cas particulier pour une marche à reconstituer, le capteur étant fixé à la cuisse de la jambe valide, la portion effective du mouvement étant accomplie par la jambe valide, une bonne solution est représentée si les variables d'état sont une vitesse angulaire et une position angulaire de la cuisse portant le capteur. Une constitution possible du dispositif est réalisée si le filtre numérique résout un système d'équations

EP 2 083 684 B1

$$\begin{cases} \hat{x}_1 = x_2 \\ \hat{x}_2 = f_1(x_1, x_2) \end{cases}$$

pour engendrer le modèle de référence du mouvement, et l'observateur numérique résout un système d'équations $\dot{z} = f_2(z, u)$ puis $x_i = f3(z, u)$ où z est le paramètre intermédiaire qui dépend de $x_i$ et $w_2$ les variables d'état, $x_i$ une des variables d'état, u le signal, du capteur (1) et $f_1$, $f_2$ et $f_3$ des fonctions.

Ces aspects de l'invention ainsi que d'autres seront maintenant décrits en liaison aux figures suivantes :

- la figure 1 illustre le schéma d'implantation du procédé,
- la figure 2 illustre le contrôleur artificiel,
- la figure 3 illustre les représentations d'un cycle de mouvements,
- la figure 4 illustre les signaux du capteur,
- la figure 5 illustre l'ajustement du modèle et des résultats expérimentaux,
- et la figure 6 donne une fonction de commande.

On passe à la description d'un mode de réalisation spécifique de l'invention pour une application particulière.

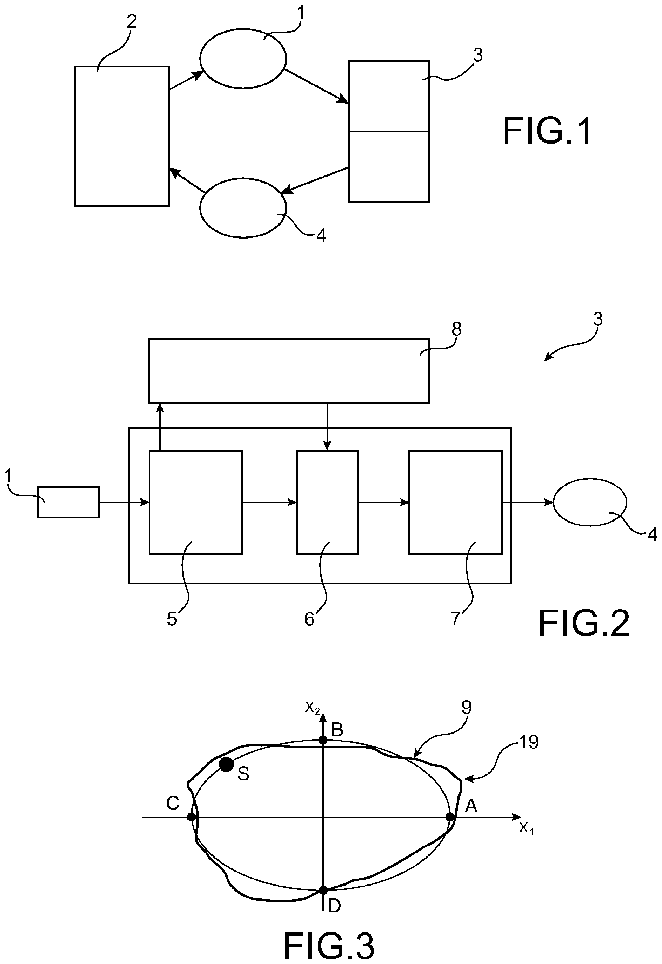

Le schéma d'implantation du procédé est résumé à la figure 1. Il comprend au moins un capteur 1, fixé sur une personne que le procédé doit aider et qui est sensible au mouvement de son système musculo-squelettique 2. Le système comprend encore un contrôleur artificiel 3 qui peut être porté aussi par la personne, et un dispositif moteur 4. Le capteur 1 peut notamment comporter des accéléromètres, inclinomètres, gyromètres, etc. mesurant une direction ou une inclinaison ; il peut être fixé à la cuisse de la jambe valide du porteur dans le cas où c'est la démarche que le procédé doit améliorer, mais d'autres capteurs peuvent être placés ailleurs, par exemple sur le tronc du porteur. Le nombre de capteurs 1 n'est pas critique, et un seul suffit souvent.

Le contrôleur artificiel 3 réagit aux mesures du capteur 1 pour calculer quel mouvement appliquer à la jambe invalide ; il estime les valeurs instantanées prises par des variables d'état du mouvement à partir des mesures du capteur 1 et élabore la commande appliquée au dispositif moteur 4.

La description porte maintenant plus précisément sur le contrôleur artificiel 3 au moyen de la figure 2. Le contrôleur artificiel 3 comprend trois modules en ligne : un module d'acquisition 5 et de numérisation du signal des capteurs 1 ; un module de calcul 6 en temps réel et un module d'élaboration de la commande 7 qui fournit les instructions de commande au dispositif moteur 4. Le contrôleur artificiel 3 peut être relié à un micro-calculateur 8. Ce dernier reçoit des signaux numérisés provenant du module d'acquisition 5 et restitue des paramètres d'observateur au module de calcul 6, de la façon qui sera décrite plus loin. Le module d'acquisition 5 reçoit des signaux analogiques du capteur 1, envoie des signaux numérisés aussi au module de calcul 6, qui lui-même fournit des valeurs instantanées des variables d'état du mouvement au module de commande 7.

Lors de la mise en oeuvre du procédé selon 1 a présente invention, on distingue deux phases principales. La première phase est une phase d'étalonnage destinée à paramétrer le module de calcul 6 en temps réel et éventuellement le module d'élaboration de la commande 7. La phase d'étalonnage se décompose en quatre étapes décrites ci-après. La seconde phase est une phase d'utilisation, au cours de laquelle le module de calcul 6 en temps réel est opérationnel.

La présente invention vise à suivre un mouvement « cyclique », telle la marche. Le signal détecté par chaque capteur utilisé doit être cyclique, ou autrement dit sensiblement périodique.

Lors d'une étape initiale de la phase d'étalonnage, on enregistre le signal mesuré par le capteur pendant une durée au moins égale à un cycle du signal détecté. On sélectionne alors une portion du signal enregistré d'une durée d'un cycle, ci-après appelé signal de référence.

On définit ensuite un modèle de type oscillateur permettant de générer un signal présentant sur un cycle donné une forme sensiblement identique au signal de référence.

Le modèle d'oscillateur $\Sigma$ utilisé dans cette réalisation de la présente invention appartient à la classe des systèmes de Lure. Plus précisément, l'oscillateur est défini à partir d'un jeu d'équations utilisant des variables d'état, selon les formules suivantes :

$$\Sigma : \begin{cases} \dot{x} = A.x + f(y) \\ y = C.x \end{cases}$$

où x est un vecteur de variables d'état, $\dot{x}$ est le vecteur dérivé de x, y un vecteur de variables de sortie, f() une fonction

3

non linéaire et A et C des matrices linéaires de paramètres.

La définition de la fonction f et des matrices linéaires de paramètres se fait par un procédé d'optimisation.

Une fois le modèle $\Sigma$ ajusté, on définit un système observateur $\Sigma$' associé au modèle $\Sigma$ selon le système d'équations suivante :

$$\Sigma' : \begin{cases} \hat{x} = A.\hat{x} + f(y) + K(\hat{y} - y) \\ \hat{y} = C.\hat{x} \end{cases}$$

Où $\hat{x}$ est une estimée du vecteur x, $\hat{y}$ est une estimée du vecteur de sortie y, et K une matrice linéaire de paramètres.

La définition de K se fait en fonction du choix de A, C et f selon un procédé connu de l'homme de l'art. Des exemples de définition sont donnés par la suite. Le système observateur diffère donc du modèle par un terme adaptatif $K(\hat{y}-y)$ qui, comme on le verra, permet de corriger la commande quand la démarche de la jambe valide diffère du modèle de façon à améliorer la coordination et la synchronisation des jambes.

On définit ensuite une fonction de commande w destinée à être mise en oeuvre par le module d'élaboration de la commande 7. Les instructions de commande D sont fonction des valeurs de variables d'état estimées $\hat{x}$ selon la formule D=w($\hat{x}$).

Une fois définis, le système observateur $\Sigma$' et la fonction de commande w sont respectivement transférés, ou autrement dit programmés, dans le module de calcul 6 en temps réel et dans le module d'élaboration de la commande 7.

Une fois cette phase d'étalonnage terminée, le micro-calculateur 8 peut être déconnecté. Le contrôleur artificiel 3 programmé peut désormais fonctionner de façon autonome.

Lors de l'utilisation du contrôleur artificiel 3, le module de calcul 6 en temps réel reçoit le signal mesuré par le capteur après numérisation. Le module de calcul 6 est un système observateur correspondant au système $\Sigma$' préalablement défini, dans lequel l'entrée y est désormais remplacée par la mesure u du capteur. Le système observateur du module de calcul 6 peut donc être écrit selon la formule suivante :

$$\Sigma' : \begin{cases} \hat{x} = A.\hat{x} + f(u) + K(\hat{y} - u) \\ \hat{y} = C.\hat{x} \end{cases}$$

Un exemple détaillé de mise en oeuvre de la présente invention est décrit ci-après.

On se reporte à la figure 3. Un mouvement peut être défini par des variables d'état ; deux (x1 et x2) peuvent suffire dans le cas de la marche, à savoir la position angulaire et la vitesse angulaire de la cuisse. Une foulée complète, correspondant à deux pas consécutifs, est un cycle du mouvement qui est représenté par une courbe de modèle 9 fermée dont chaque point, ou chaque phase de la foulée, est défini par des valeurs instantanées de x1 et x2. La courbe de modèle 9 correspond au cycle que décriraient les variables d'état estimées $\hat{x}$ par le module de calcul 6 en temps réel si le signal de mesure u reçu par ce dernier correspondait au signal de référence défini ci-dessus.

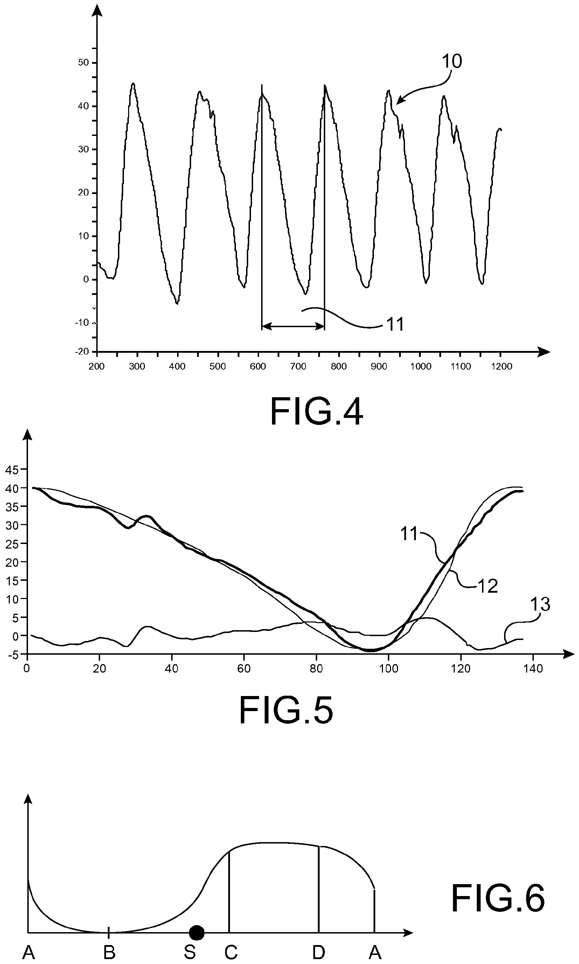

Dans une marche réelle, chacune des foulées sera différente, mais en s'écartant de petites quantités seulement du modèle. Le signal du capteur 1 est à peu près périodique et pourra prendre l'aspect représenté à la figure 4, où une courbe de mesure 10 donne son intensité en fonction du temps.

La description porte maintenant plus précisément sur le module de calcul 6. Il sert à convertir les signaux issus du capteur 1 et mis en forme par le module d'acquisition 5 en variables d'état du modèle retenu pour la marche.

Le signal de du capteur 1 est numérisé par le module 5 avec une fréquence d'échantillonnage donnée. Le module 5 fournit une succession, ou suite, d'échantillons de mesure u(n), où n correspond à un instant donné. Pour chaque échantillon u(n), le module de calcul 6 détermine des valeurs $\hat{x}$(n) des variables d'état estimées. Chaque valeur $\hat{x}$(n) n'est pas uniquement fonction de l'échantillon u(n) présent mais également de l'historique des échantillons précédents.

En se référant à la figure 3, chaque valeur $\hat{x}$(n) des variables d'état estimées peut être représentée par un point du plan. Une courbe 19 représente un exemple de suite de valeurs de variables d'état estimées obtenue pour une foulée « imparfaite ». Le système $\Sigma$' permet d'assurer une convergence de la courbe 19 vers la courbe modèle 9.

Dans le cas particulier de l'invention appliquée à une marche, l'élément $\Sigma$ peut être défini au moyen du système d'équations (3) :

$$\sum \begin{cases} \dot{x}_1 = x_2 \\ \dot{x}_2 = \mu(1 - bx_1 - x_1^2)x_2 - \omega_0^2 x_1 \\ y = x_1 \end{cases}$$

où $\mu$, b et $\omega0$ sont réglés de façon optimale par le micro-calculateur 8 pour minimiser l'erreur entre les résultats de calculs et les mesures pendant l'étape préliminaire d'étalonnage. La figure 5 donne, en superposition au profil de référence 11 représenté à la figure 4, mais avec une échelle différente, la courbe de la sortie (y) 12 de l'élément $\sum$ et la courbe d'erreur 13 entre les courbes 11 et 12.

Afin de déterminer le système $\sum'$, considérons la variable z définie par l'équation (4) $z = x_2 + k_1.y + k_2.y^2 + k_3.y^3$. On obtient l'équation (5) en exploitant le système d'équations (3) :

$$\dot{z} = (\mu + k_1)x_2 + (2.k_2 - \mu.b)x_1.x_2 + (3.k_3 - \mu)x_1^2 - x_2 - \omega_0^2.x_1$$

En choisissant $k_1 = -\mu - 1$, $k_2 = \mu.b/2$ et $k_3 = \mu/3$, on obtient l'équation (6) :

$$\dot{z} = -z + (k_1 - \omega_0^2)y + k_2.y^2 + k_3.y^3$$

qui, avec le système d'équations (7) :

$$\begin{cases} \hat{x}_1 = y \\ \hat{x}_2 = z - k_1.y - k_2.y^2 - k_3.y^3 \\ \hat{y} = \hat{x}_1 \end{cases}$$

correspond au système observateur $\sum'$. Les coefficients $k_1$, $k_2$ et $k_3$ sont obtenus à partir des valeurs de b, $\mu$ et $\omega_0$ calculées par l'élément $\sum$ à l'étape d'étalonnage.

Lors de l'utilisation du contrôleur artificiel 3, le module de calcul 6 reçoit le signal de mesure u. Le module de calcul 6 est alors un système observateur correspondant au système $\sum'$ préalablement défini, dans lequel l'entrée y est désormais remplacée par la mesure u du capteur.

L'élément $\sum'$ recalcule sans cesse la valeur de z après avoir calculé $\dot{z}$ selon l'équation (6), puis $\hat{x}_2$ d'après la seconde équation du système (7) et délivre en sortie des valeurs des variables d'état $\hat{x}(\hat{x}_1, \hat{x}_2)$.

Dans le cas présent, $\hat{x}_2$ correspond à une estimation de la vitesse angulaire et $\hat{x}_1$ à une estimation de la position angulaire.

Nous soulignons que l'élément $\sum$, utilisé pendant l'étape d'étalonnage pour donner les valeurs des coefficients et construire le modèle donnant les relations entre les variables d'état, reste inactif pendant l'utilisation de l'invention où seul l'élément $\sum'$ travaille, alors que ce dernier était inerte pendant l'étalonnage. Le micro-calculateur 8 est d'ailleurs retiré pendant cette utilisation, après avoir fourni les résultats au contrôleur artificiel 3.

L'élément $\sum$ sert à décrire le mouvement, l'élément $\sum'$ à synchroniser la commande sur lui.

Tel est le système par lequel les variables d'état du mouvement sont calculées de façon continue, avec une bonne synchronisation avec les indications du capteur 1. Il faut remarquer qu'une reconstitution parfaite des variables d'état serait obtenue seulement dans le cas où la démarche correspondrait exactement à la courbe modèle 9 pour chacune des foulées, ce qui n'est pas le cas en réalité, mais un autre effet de l'élément d'observation $\sum'$ est d'atténuer les erreurs provenant de cette différence entre les foulées réelles et la foulée modélisée.

Le module de commande 7 délivre des instructions de commande C en fonction des variables d'état estimées $\hat{x}(\hat{x}_1, \hat{x}_2)$. Selon des formules telles que $D = w(\hat{x})$. La fonction w dépend entre autres du dispositif moteur 4 placé sur le membre invalide et de la nature de commande qu'il faut lui appliquer.

Afin de définir le type de fonction w, pouvant s'appliquer pour des personnes hémiplégiques, on peut par exemple utiliser le protocole expérimental suivant : on place sur une personne valide (autre que la personne invalide que l'on souhaite par la suite aider) un capteur de mouvement sur un de ses membres correspondant au membre de la personne invalide sur lequel on va placer ultérieurement le capteur de mouvement ainsi qu'un capteur d'activité musculaire sur l'autre de ses membres. On observe alors au moins une foulée de la personne valide. Pour un cycle de mesure, on établit une correspondance entre les valeurs de variable d'état estimées (à partir du signal de mesure correspondant) et les mesures d'activités musculaires relevées par le capteur d'activité musculaire. Par ailleurs, on peut définir une correspondance entre chaque mesure d'activité musculaire du membre valide de la personne valide et une valeur de paramètres de stimulation du membre invalide de la personne invalide permettant de retrouver la mesure d'activité musculaire correspondante. Ainsi, on peut définir la fonction w, par exemple, sous la forme d'une table de correspondance associant à chaque valeur de variable d'état estimée $\hat{x}$ une commande correspondant aux paramètres de stimulation susmentionnés.

La commande se fait d'après chaque phase du cycle. La figure 6 donne un exemple concret d'après le cycle de la figure 3, pour des points correspondant à A, B, C ou D de ce groupe et un point quelconque S.

Par ailleurs, afin de tenir compte des différences entres les individus, la fonction w susmentionnée pourra être paramétrée de façon à pouvoir régler notamment l'amplitude des mouvements de la personne invalide. La définition de tels paramètres pourra alors se faire lors de la phase d'étalonnage.

Selon une variante de réalisation du dispositif décrit ci-dessus et représenté en figure 2, le contrôleur artificiel 3 ne contient pas de module de numérisation. Le signal reçu par le module de calcul 6 en temps réel est alors analogique. Le module de calcul 6 et le module 7 sont alors des dispositifs analogiques.

Les avantages principaux de l'invention sont donc le caractère continu de la commande qui aboutit à une synchronisation bien meilleure, la stabilité de l'oscillateur et la facilité de mise en oeuvre.

Le procédé a été décrit dans le cas d'une personne invalide, et plus particulièrement une personne hémiplégique. D'autres applications sont possibles.

On pourra par exemple commander un robot, éventuellement par télé-opération, à partir des commandes émises par le module 7. Ce robot peut par exemple suivre la personne sur laquelle est placé le capteur 1 afin de l'assister dans ces mouvements.

## Revendications

1. Dispositif de suivi de mouvement d'un être vivant, comprenant un capteur fixé à l'être vivant et recueillant des signaux représentatifs de son mouvement, et un contrôleur artificiel (3) de traitement des signaux du capteur, où le contrôleur artificiel (3) comprend un module de calcul (6), **caractérisé en ce que** :

   - le module de calcul (6) comprend un observateur numérique apte à délivrer un signal de sortie d'après un modèle de référence du mouvement enregistré dans le contrôleur artificiel (3), le signal de sortie étant fonction des signaux du capteur et du modèle de référence,
   - le modèle de référence étant un modèle défini au cours d'une étape d'étalonnage et de type oscillateur,
   - le modèle de référence et le signal de sortie étant exprimés en fonction des variables d'état du mouvement relatives à une phase du mouvement.

2. Dispositif de suivi de mouvement selon la revendication 1, **caractérisé en ce qu'**il comprend un calculateur (8) d'étalonnage, délivrant les variables d'état du modèle de référence au module de calcul (6) en fonction d'au moins une série préliminaire de signaux du capteur.

3. Dispositif de suivi du mouvement selon la revendication 2, **caractérisé en ce que** le calculateur (8) est séparable du contrôleur artificiel (3).

4. Dispositif de suivi du mouvement selon l'une quelconque des revendications 2 ou 3, **caractérisé en ce que** le calculateur comporte un filtre numérique de la série préliminaire des signaux du capteur.

5. Dispositif de suivi du mouvement selon la revendication 4, **caractérisé en ce que** le filtre numérique est conforme à un oscillateur selon un système de Lure.

6. Dispositif de suivi du mouvement selon l'une quelconque des revendications 1 à 5, **caractérisé en ce que** le capteur (1) mesure une vitesse angulaire et une position angulaire.

7. Dispositif de suivi du mouvement selon l'une quelconque des revendications 1 à 6, **caractérisé en ce qu'**il comprend un module d'élaboration d'une commande (7) pour compléter ou reproduire le mouvement par un dispositif moteur.

8. Procédé de suivi du mouvement d'un être vivant, consistant à mesurer continûment une portion effective du mouvement par un capteur fixé sur l'être vivant, à utiliser des signaux du capteur de façon à estimer des valeurs de variables d'état du mouvement pour produire des instructions de commande et entraîner un dispositif accomplissant une portion complémentaire du mouvement, **caractérisé en ce qu'**il consiste à utiliser aussi un modèle de référence de type oscillateur, obtenu dans une étape d'étalonnage et défini par des séries de valeurs prises par les variables d'état du mouvement à des phases du mouvement, et un observateur numérique, associé au modèle de référence pour estimer un signal de sortie et pour estimer les valeurs des variables d'état du mouvement.

9. Procédé de suivi de mouvement selon la revendication 8, **caractérisé en ce que** les variables d'état comprennent deux variables d'état $(x_1, x_2)$ déductibles l'une de l'autre par une dérivation temporelle.

10. Procédé de suivi de mouvement selon la revendication 9, **caractérisé en ce que** les variables d'état représentent une position et une vitesse.

11. Procédé de suivi de mouvement selon la revendication 8, caractérisé en ce l'observateur numérique ($\Sigma$') effectue continûment un ajustement d'un paramètre intermédiaire $(z)$ d'après des mesures $(u)$ du capteur (1) en résolvant numériquement une équation différentielle (6), et un ajustement d'au moins une des variables d'état $(\hat{x}_2)$ d'après le paramètre intermédiaire.

12. Procédé de suivi de mouvement selon l'une quelconque des revendications 8 à 11, **caractérisé en ce que** le mouvement est une marche.

13. Procédé de suivi de mouvement selon les revendications 10 et 12, **caractérisé en ce que** les variables d'état sont une vitesse angulaire et une position angulaire d'une cuisse portant le capteur (1).

14. Procédé d'aide au mouvement selon les revendications 11 et 13, **caractérisé en ce que** le modèle du mouvement est défini par un filtre numérique ($\Sigma$) qui résout un système d'équations

$$\begin{cases} \dot{\hat{x}}_1 = x_2 \\ \dot{\hat{x}}_2 = f_1 (x_1, x_2) \end{cases}$$

pour engendrer le modèle de référence du mouvement, et l'observateur numérique ($\Sigma$') résout un problème d'équations $\dot{z} = f_2(z, u)$ puis $xi = f3 (z, u)$ où $z$ est le paramètre intermédiaire, qui dépend de $x_i$, $x_1$ et $x_2$ sont les variables d'état, $x_i$ une des variables d'état, u le signal du capteur (1) et $f_1$, $f_2$ et $f_3$ des fonctions.

## Patentansprüche

1. Vorrichtung zum Verfolgen der Bewegung eines Lebewesens, enthaltend einen Sensor, der an das Lebewesen befestigt ist und für seine Bewegung repräsentative Signale empfängt, sowie einen künstlichen Controller (3) zum Verarbeiten der Signale des Sensors, wobei der künstliche Controller (3) ein Rechenmodul (6) enthält, **dadurch gekennzeichnet, dass**:

   - das Rechenmodul (6) einen digitalen Beobachter enthält, der dazu geeignet ist, ein Ausgangssignal gemäß einem Referenzmodell der in dem künstlichen Controller (3) aufgezeichneten Bewegung auszugeben, wobei das Ausgangssignal Funktion von den Signalen des Sensors und des Referenzmodells ist,
   - wobei das Referenzmodell ein im Laufe eines Kalibrierungsschritts definiertes Modell und vom Typ Oszillator ist,
   - wobei das Referenzmodell und das Ausgangssignal in Abhängigkeit von eine Bewegungsphase betreffenden Zustandsvariablen der Bewegung ausgedrückt werden.

2. Bewegungsverfolgungsvorrichtung nach Anspruch 1, **dadurch gekennzeichnet, dass** sie einen Kalibrierungsrechner (8) enthält, der die Zustandsvariablen des Referenzmodells in Abhängigkeit von zumindest einer einleitenden Reihe von Signalen des Sensors an das Rechenmodul (6) liefert.

3. Bewegungsverfolgungsvorrichtung nach Anspruch 2, **dadurch gekennzeichnet, dass** der Rechner (8) von dem künstlichen Controller (3) getrennt werden kann.

4. Bewegungsverfolgungsvorrichtung nach einem der Ansprüche 2 oder 3, **dadurch gekennzeichnet, dass** der Rechner ein digitales Filter für die einleitende Reihe von Signalen des Sensors enthält.

5. Bewegungsverfolgungsvorrichtung nach Anspruch 4, **dadurch gekennzeichnet, dass** das digitale Filter einem Oszillator nach einem Lure-System entspricht.

6. Bewegungsverfolgungsvorrichtung nach einem der Ansprüche 1 bis 5, **dadurch gekennzeichnet, dass** der Sensor (1) eine Winkelgeschwindigkeit und eine Winkelposition misst.

7. Bewegungsverfolgungsvorrichtung nach einem der Ansprüche 1 bis 6, **dadurch gekennzeichnet, dass** sie ein Modul zum Ausarbeiten eines Steuerbefehls (7) zum Vervollständigen bzw. Wiedergeben der Bewegung durch eine Antriebsvorrichtung enthält.

8. Verfahren zum Verfolgen der Bewegung eines Lebewesens, das darin besteht, kontinuierlich einen effektiven Abschnitt der Bewegung über einen Sensor zu messen, der an das Lebewesen befestigt ist, und Signale des Sensors so zu verwenden, dass Werte von Zustandsvariablen der Bewegung geschätzt werden, um Steueranweisungen zu erzeugen und eine Vorrichtung zum Ausführen eines komplementären Abschnitts der Bewegung anzutreiben, **dadurch gekennzeichnet, dass** es darin besteht, auch ein Referenzmodell vom Typ Oszillator zu verwenden, das in einem Kalibrierungsschritt erhalten wird durch Reihen von Werten definiert ist, die von den Zustandsvariablen der Bewegung zu Bewegungsphasen aufgenommen werden, sowie einen digitalen Beobachter, der dem Referenzmodell zugeordnet ist, um ein Ausgangssignal zu schätzen und die Werte der Zustandsvariablen der Bewegung zu schätzen.

9. Bewegungsverfolgungsverfahren nach Anspruch 8, **dadurch gekennzeichnet, dass** die Zustandsvariablen zwei Zustandsvariablen ($x_1$, $x_2$) umfassen, die durch zeitliche Ableitung voneinander abgeleitet werden können.

10. Bewegungsverfolgungsverfahren nach Anspruch 9, **dadurch gekennzeichnet, dass** die Zustandsvariablen eine Position und eine Geschwindigkeit darstellen.

11. Bewegungsverfolgungsverfahren nach Anspruch 8, **dadurch gekennzeichnet, dass** der digitale Beobachter ($\Sigma$') kontinuierlich eine Anpassung eines Zwischenparameters (z) gemäß der Messungen (u) des Sensors (1) durchführt, indem eine Differentialgleichung (6) numerisch gelöst wird, sowie eine Anpassung zumindest einer der Zustandsvariablen ($\hat{x}_2$) gemäß des Zwischenparameters.

12. Bewegungsverfolgungsverfahren nach einem der Ansprüche 8 bis 11, **dadurch gekennzeichnet, dass** die Bewegung ein Gehen ist.

13. Bewegungsverfolgungsverfahren nach einem der Ansprüche 10 und 12, **dadurch gekennzeichnet, dass** die Zustandsvariablen eine Winkelgeschwindigkeit und eine Winkelposition eines den Sensor (1) tragenden Oberschenkels ist.

14. Bewegungsunterstützungsverfahren nach einem der Ansprüche 11 und 13, **dadurch gekennzeichnet, dass** das Bewegungsmodell über ein digitales Filter ($\Sigma$) definiert wird, welches ein System aus Gleichungen

$$\begin{cases} \hat{x}_1 = x_2 \\ \hat{x}_2 = f_1\,(x_1, x_2) \end{cases}$$

löst, um das Referenzmodell der Bewegung zu erzeugen, wobei der digitale Beobachter ($\Sigma$') eine Aufgabenstellung aus Gleichungen $\dot{z} = f_2\,(z, u)$, dann $x_i = f_3\,(z, u)$ löst, worin z der Zwischenparameter ist, der von $x_1$ abhängt, $x_1$ und $x_2$ Zustandsvariablen sind, $x_i$ eine der Zustandsvariablen ist, u des Signal des Sensors (1) ist und $f_1$, $f_2$ und $f_3$ Funktionen sind.

### Claims

1. A device for following movement of a living being, comprising a sensor attached to the living being and collecting signals representative of the movement thereof, and an artificial controller (3) for processing signals from the sensor, wherein the artificial controller (3) comprises a computation module (6), **characterized in that**:

- the computation module (6) comprises a digital observer capable of delivering an output signal according to a reference model of the movement recorded in the artificial controller (3), the output signal being a function of the signals from the sensor and of the reference model,
- the reference model being a model defined in a calibration step and of an oscillator type,
- the reference model and the output signal being expressed as state variables of the movement relative to a phase of the movement.

2. The device for following movement according to claim 1, **characterized in that** it comprises a calibration computer (8), delivering the state variables of the reference model to the computation module (6) depending at least on a preliminary series of signals from the sensor.

3. The device for following the movement according to claim 2, **characterized in that** the computer (8) is separable from the artificial controller (3).

4. The device for following the movement according to claim 2 or 3, **characterized in that** the computer includes a digital filter for the preliminary series of signals from the sensor.

5. The device for following the movement according to claim 4, **characterized in that** the digital filter is compliant with an oscillator according to a Lure system.

6. The device for following the movement according to any of claims 1 to 5, **characterized in that** the sensor (1) measures an angular velocity and an angular position.

7. The device for following the movement according to any of claims 1 to 6, **characterized in that** it comprises a module for elaborating a control (7) for completing or reproducing the movement by means of a driving device.

8. A method for following the movement of a living being, consisting of continuously measuring an effective portion of the movement by means of a sensor attached on the living being, of using signals from the sensor in order to estimate values of state variables of the movement in order to produce control instructions and to drive a device accomplishing a complementary portion of the movement, **characterized in that** it consists in also using a reference model of oscillator type, obtained in a calibration step and defined by series of values taken by the state variables of the movement and a digital observer, associated to the reference model in phases of the movement, in order to estimate an output signal and to estimate the values of the state variables of the movement.

9. The method for following movement according to claim 8, **characterized in that** the state variables comprise two state variables ($x_1$, $x_2$) which may be inferred from each other by time derivation.

10. The method for following movement according to claim 9, **characterized in that** the state variables represent a position and a velocity.

11. The method for following movement according to claim 8, **characterized in that** the digital observer ($\Sigma'$) continuously carries out an adjustment of an intermediate parameter (z) according to measurements (u) of the sensor (1) by digitally solving a differential equation (6) and an adjustment of at least one of the state variables ($\hat{x}_2$) according to the intermediate parameter.

12. The method for following movement according to any of claims 8 to 11, **characterized in that** the movement is walking.

13. The method for following movement according to claims 10 and 12, **characterized in that** the state variables are an angular velocity and an angular position of a thigh bearing the sensor (1).

14. The method for assisting movement according to claims 11 and 13, **characterized in that** the model of the movement is defined by a digital filter ($\Sigma$) which solves a system of equations

$$\begin{cases} \hat{x}_1 = x_2 \\ \hat{x}_2 = f_1 (x_1, x_2) \end{cases}$$

in order to generate the reference model of the movement, and the digital observer ($\Sigma$) solves a system of equations $\dot{z} = f_2(z, u)$ and then $x_i = f3 (z, u)$ wherein z is the intermediate parameter which depends on $x_i$, $x_1$ and $x_2$ the state variables, $x_i$ being one of the state variables, u the signal from the sensor (1) and $f_1$, $f_2$ and $f_3$ being functions.

$$\hat{x}_1 = x_2$$

$$\hat{x}_2$$

EP 2 083 684 B1

FIG.1

FIG.2

FIG.3

11

FIG.4

FIG.5

FIG.6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2004088057 A

**Littérature non-brevet citée dans la description**

- **WILLIAMSON, R.P. ; ANDREWS, B.J. ; AU, R.** Control of neural prostheses. II. Event détection using machine learning ''Proceedings of the RESNA. *96 Annual Conférence Exploring New Horizons... Pioneering the 21st Century,* 1996, 291-3

- **PAPPAS I.P.I. ; KELLER, T. ; MANGOLD, S.** A reliable, gyroscope base gait phase détection sensor embedded in a shoe insole. *Proceedings of IEEE Sensors 002. First IEEE International Conférence on Sensors,* 2002, vol. 2, 1085-8
- Drift-free attitude estimation using quasi-linear observers. **REHBINDER H et al.** NEW DIRECTIONS AND APPLICATIONS IN CONTROL THEORY. SPRINGER-VERLAG, 2005, 321-336